# FASCICULE DE BREVET EUROPEEN

(11) **EP 1 325 732 B1**
(45) Date de publication et mention de la délivrance du brevet: **17.09.2008**
(21) Numéro de dépôt: 02293072.1
(22) Date de dépôt: 12.12.2002
(51) Int. Cl.: A61Q 5/06, A61K 8/04, A61K 8/33, A61K 8/85

(54) **Laque capillaire à base de polyesters sulfoniques ramifiés et à haute teneur en eau**
Haarspray auf Basis von verzweigten Sulfonsäure-Polyestern mit erhöhtem Wassergehalt
Hair spray based on branched sulfonic polyesters with high water content

(30) Priorité: 17.12.2001 FR 0116318
(43) Date de publication de la demande: 09.07.2003
(73) Titulaire: L'ORÉAL, 75008 Paris (FR)
(72) Inventeur: Rollat-Corvol, Isabelle, 75017 Paris (FR); Dupuis, Christine, 75018 Paris (FR); Benabdillah, Katarina, 92110 Clichy (FR); Condamine, Christine, 95610 Eragny s/Oise (FR)
(74) Mandataire: Casalonga, Axel

(56) Documents cités:
- EP-A- 0 524 346
- EP-A- 0 574 607
- DE-A- 19 943 430
- US-A- 4 525 524

## Description

La présente invention concerne des compositions de coiffage conditionnées dans un dispositif aérosol comprenant un polyester sulfonique ramifié et une proportion importante en eau, ainsi qu'un procédé de coiffage utilisant une telle composition.

L'utilisation de polyesters sulfoniques ramifiés dans des compositions de coiffage et de fixation des cheveux est connue et décrite par exemple dans les documents EP 0 966 946, WO 98/38969, WO 99/63955 et DE 199 43 430.

Toutefois, la demanderesse a constaté que l'utilisation de ces polyesters sulfoniques ramifiés dans des laques capillaires contenant des proportions importantes en alcool donnait, certes, d'excellentes propriétés coiffantes mais ne permettait pas d'obtenir un pouvoir laquant satisfaisant.

Par ailleurs, les polyesters sulfoniques ramifiés appliqués sous forme de laques à forte teneur en alcool laissent, après élimination par brossage, les cheveux avec un toucher sec. Ce phénomène indésirable est particulièrement visible pour des cheveux teints.

Le brevet US 4,525,524 divulgue des compositions de polyesters sulfoniques linéaires pulvérisables sans agent propulseur, pouvant être utilisées pour la fixation des cheveux.

La demanderesse a constaté que l'augmentation de la teneur globale en eau des laques capillaires améliorait non seulement le pouvoir laquant des compositions obtenues, mais conférait également aux cheveux, après brossage, un toucher plus lisse et plus cosmétique que les laques connues à forte teneur en alcool.

L'invention a par conséquent pour objet une composition de coiffage conditionnée dans un dispositif aérosol comprenant
- de 45 à 65% en poids d'une phase liquide contenant, à l'état dissous ou finement dispersé dans un milieu liquide aqueux ou hydroalcoolique cosmétiquement acceptable, au moins un polyester sulfonique ramifié et,
- de 35 à 55 % en poids de diméthyléther en tant qu'agent propulseur,
la teneur totale en eau de la phase liquide étant comprise entre 65 % et 99 % en poids.

L'invention a également pour objet un procédé de coiffage par pulvérisation d'une telle composition de coiffage, conditionnée dans un dispositif aérosol, sur les cheveux.

Comme indiqué ci-dessus, la phase liquide de la composition de coiffage de la présente invention contient un milieu aqueux ou hydroalcoolique, à savoir un mélange d'eau et d'éthanol.
Lorsqu'elle contient de l'éthanol, le rapport en poids de l'éthanol présent dans la phase liquide au diméthyléther (agent propulseur) est de préférence inférieur ou égal à 1.
Dans un mode de réalisation préféré, la teneur en éthanol de la phase liquide est inférieure ou égale à 35 % en poids, de préférence inférieure ou égale à 25 % en poids, et en particulier inférieure ou égale à 15 % en poids.

Les polyesters sulfoniques ramifiés utilisés dans les compositions de la présente invention sont connus dans la technique. Leur structure et synthèse sont décrites dans les documents WO 95/18191, WO 97/08261 et WO 97/20899.

On préfère en particulier des polyesters sulfoniques ramifiés obtenus par polycondensation
(a) d'au moins un acide dicarboxylique ne portant pas de fonction sulfonique,
(b) d'au moins un diol ou d'un mélange d'un diol et d'une diamine,
(c) d'au moins un monomère comportant deux fonctions réactives, identiques ou différentes, choisies parmi les groupes hydroxyle, amino et carboxyle, et portant en outre au moins une fonction sulfonique, et
(d) d'au moins un monomère comportant au moins trois fonctions réactives, identiques ou différentes, choisies parmi les groupes hydroxyle, amino et carboxyle.

Les acides dicarboxyliques formant les motifs (a) peuvent être des acides dicarboxyliques aliphatiques, des acides dicarboxyliques alicycliques, des acides dicarboxyliques aromatiques et des mélanges de tels acides.
On peut citer à titre d'exemples l'acide 1,4-cyclohexanedioïque, l'acide succinique, l'acide glutarique, l'acide adipique, l'acide azélaïque, l'acide sébacique, l'acide fumarique, l'acide maléique, l'acide 1,3-cyclohexandioïque, l'acide phtalique, l'acide téréphtalique et l'acide isophtalique et un mélange de tels acides.

Les diols formant les motifs (b) sont choisis par exemple parmi les alcanediols et les polyalkylènediols, et on peut citer à titre d'exemple l'éthylèneglycol, le propylèneglycol, le diéthylèneglycol, le triéthylèneglycol et le polypropylèneglycol.

Les diamines susceptibles de former une partie des motifs (b) sont de préférence choisies parmi les alcanediamines et les poly(oxyalkylène)diamines.

Le terme "fonction sulfonique" des motifs (c) englobe à la fois la fonction acide sulfonique (-SO₃H) et les fonctions salifiées correspondantes obtenues par neutralisation de la fonction acide sulfonique avec une base, par exemple avec un hydroxyde de métal alcalin.
Les fonctions sulfoniques sont de préférence sous forme neutralisée par une base organique ou minérale.
Les motifs (c) sont dérivés par exemple d'acides dicarboxyliques, d'esters d'acides dicarboxyliques, de glycols et d'hydroxyacides, portant tous au moins un groupe sulfonique, sous forme acide et/ou neutralisée, de préférence sous forme neutralisée.
Les motifs (c) portant au moins une fonction sulfonique représentent de préférence de 2 à 15 % en moles de l'ensemble des monomères.

Les motifs (d) dérivés de monomères multifonctionnels sont présents de préférence en une quantité comprise entre 0,1 et 40 % en moles rapportée à l'ensemble des monomères.
Les monomères multifonctionnels formant les motifs (d) sont choisis par exemple parmi le triméthyloléthane, le triméthylolpropane, le glycérol, le pentaérythritol, le sorbitol, l'anhydride trimellitique, l'érythritol, le thréitol, le dipentaérythritol, le dianhydride pyromellitique et l'acide diméthylpropionique.

Les polyesters sulfoniques ramifiés peuvent comporter, en plus des quatre types de motifs (a) à (d) décrits ci-dessus, des motifs (e) dérivés de monomères comportant deux fonctions réactives différentes, choisis par exemple parmi les hydroxyacides carboxyliques et les aminoacides carboxyliques ou un mélange de ceux-ci.
Ces motifs (e) peuvent représenter jusqu'à 40 % en moles de l'ensemble des monomères (a), (b), (c), (d) et (e).

Bien entendu, les polymères sulfoniques ramifiés utilisés dans la présente invention sont obtenus de préférence à partir d'un mélange de monomères dans lequel le nombre d'équivalents de fonctions acide carboxylique est substantiellement égal au nombre d'équivalents de fonctions hydroxyle et de fonctions amino, éventuellement présentes.
Les polyesters sulfoniques ramifiés ont par conséquent une masse moléculaire relativement élevée. Leur masse moléculaire moyenne en nombre (Mₙ), déterminée par chromatographie d'exclusion par la taille (SEC), est de préférence supérieure à 5000 g/mole. Leur indice de polydispersité est supérieur à 3, de préférence d'environ 4.

Les polymères sulfoniques ramifiés utilisés dans les compositions de coiffage de la présente invention sont connus et commercialisés par exemple par la société Eastman. On peut citer à titre de produit commercial préféré le produit vendu sous la dénomination AQ 1350® par la société Eastman.

Les compositions de coiffage de la présente invention ont de préférence une teneur en eau dans la phase liquide comprise entre 70 et 98 % en poids, de préférence entre 75 et 96 % en poids.
La phase liquide peut contenir des composés hydrocarbonés hydroxylés autres que l'éthanol, comme par exemple le glycérol et l'isopropanol.

La phase liquide, également appelée "jus", des compositions de coiffage de la présente invention doit avoir une concentration minimale en polyester sulfonique ramifié permettant le dépôt d'une quantité suffisante de polymère permettant la fixation des cheveux.
D'une manière générale, il s'est avéré qu'une concentration de polyester sulfonique ramifié comprise entre 0,2 et 15 % rapportée au poids total de la phase liquide de la composition de coiffage, convenait bien à l'invention. On préfère en particulier une gamme de concentration allant de 0,5 à 10 % du poids de la phase liquide.

Comme indiqué ci-dessus, l'agent propulseur, à savoir le diméthyléther, représente de 35 à 55 % du poids total de la composition coiffante aérosol de la présente invention.

Les compositions de coiffage de la présente invention peuvent contenir - en plus d'un ou de plusieurs polyesters ramifiés - un ou plusieurs autres polymères fixants filmogènes connus anioniques, non ioniques, amphotères ou cationiques à l'état dissous ou dispersé (latex).

Les compositions de coiffage de la présente invention peuvent contenir en outre un ou plusieurs additifs cosmétiques ou de formulation utilisés habituellement dans le domaine cosmétique. On peut citer à titre d'exemples de tels additifs les filtres UV, les parfums, les agents conservateurs, les pigments et colorants, les agents solubilisants, les agents anti-mousse, les vitamines, les agents conditionneurs tels que des silicones solubles, dispersées ou microdispersées, les particules organiques ou minérales, synthétiques ou non, ou les agents tensioactifs.

Les compositions de coiffage de la présente invention sont de préférence des laques pour cheveux.

### Exemple

On prépare une laque capillaire A à forte teneur en eau selon l'invention et une laque capillaire B comparative à faible teneur en eau ayant les compositions suivantes :

| **Ingrédients** | **Laque A (selon l'invention)** | **Laque B (comparative)** |
|---|---|---|
| polyester sulfonique ramifié AQ 1350 (Eastman) | 3,5 % en poids de matière active (m.a) (soit 5,4 % en poids de m.a. rapporté à la phase liquide) | 3,5 % en poids de matière active (soit 5,4 % en poids rapporté à la phase liquide) |
| Eau | 41,5 % en poids (soit 63,8 % rapporté à la phase liquide) | 26,5 % en poids (soit 40,8 % rapporté à la phase liquide) |
| Ethanol | 20 % en poids (soit 30,8 % rapporté à la phase liquide) | 35 % en poids (soit 53,8 % rapporté à la phase liquide |
| Diméthyléther | 35 % en poids | 35 % en poids |

Les deux laques sont pulvérisées sur des modèles. Chaque composition est pulvérisée en comparatif sur une demie-tête. Les résultats sont jugés par évaluation sensorielle par un expert entraîné.
Le pouvoir laquant est plus important pour la laque A selon l'invention que pour la laque B comparative. Les cheveux après brossage sont plus lisses et moins secs pour la laque A selon l'invention.

## Revendications

1. Composition de coiffage conditionnée dans un dispositif aérosol comprenant
• de 45 à 65 % en poids d'une phase liquide contenant, à l'état dissous ou finement dispersé dans un milieu liquide aqueux ou hydroalcoolique cosmétiquement acceptable, au moins un polyester sulfonique ramifié, et
• de 35 à 55 % en poids de diméthyléther en tant qu'agent propulseur, la teneur totale en eau de la phase liquide étant comprise entre 65 % et 99 % en poids.

2. Composition de coiffage selon la revendication 1, **caractérisée par le fait qu'**elle contient de l'éthanol et que le rapport pondéral éthanol/diméthyléther est inférieur ou égal à 1.

3. Composition de coiffage selon la revendication 2, **caractérisée par le fait que** la phase liquide a une teneur en éthanol inférieure ou égale à 35 % en poids, de préférence inférieure ou égale à 25 % en poids, et en particulier inférieure ou égale à 15 % en poids.

4. Composition de coiffage selon l'une quelconque des revendications précédentes, **caractérisée par le fait que** le polyester sulfonique ramifié est un polymère formé par polycondensation
(a) d'au moins un acide dicarboxylique ne portant pas de fonction sulfonique,
(b) d'au moins un diol ou d'un mélange d'un diol et d'une diamine,
(c) d'au moins un monomère comportant deux fonctions réactives, identiques ou différentes, choisies parmi les groupes hydroxyle, amino et carboxyle, et portant en outre au moins une fonction sulfonique, et
(d) d'au moins un monomère comportant au moins trois fonctions réactives, identiques ou différentes, choisies parmi les groupes hydroxyle, amino et carboxyle.

5. Composition de coiffage selon la revendication 4, **caractérisée par le fait que** le polyester sulfonique ramifié contient en outre des motifs dérivés de monomères difonctionnels (e) choisis parmi les hydroxyacides carboxyliques et les aminoacides carboxyliques ou un mélange de ceux-ci.

6. Composition de coiffage selon la revendication 5, **caractérisée par le fait que** le monomère difonctionnel (e) représente jusqu'à 40 % en moles de l'ensemble des monomères.

7. Composition de coiffage selon l'une des revendications 4 à 6, **caractérisée par le fait que** le monomère (c) portant au moins une fonction sulfonique représente de 2 à 15 % en moles de l'ensemble des monomères.

8. Composition de coiffage selon l'une quelconque des revendications 4 à 7, **caractérisée par le fait que** le monomère (d) comportant au moins trois fonctions réactives représente de 0,1 à 40 % en moles de l'ensemble des monomères.

9. Composition de coiffage selon l'une quelconque des revendications 4 à 8, **caractérisée par le fait que** les acides dicarboxyliques ne portant pas de fonction sulfonique formant les motifs (a) sont choisis parmi les acides dicarboxyliques aliphatiques, les acides dicarboxyliques alicycliques, les acides dicarboxyliques aromatiques et les mélanges de tels acides.

10. Composition de coiffage selon la revendication 9, **caractérisée par le fait que** les acides dicarboxyliques formant les motifs (a) sont choisis dans le groupe formé par l'acide 1,4-cyclohexanedioïque, l'acide succinique, l'acide glutarique, l'acide adipique, l'acide azélaïque, l'acide sébacique, l'acide fumarique, l'acide maléique, l'acide 1,3-cyclohexandioïque, l'acide phtalique, l'acide téréphtalique et l'acide isophtalique et un mélange de tels acides.

11. Composition de coiffage selon l'une quelconque des revendications 4 à 10, **caractérisée par le fait que** les diols formant les motifs (b) sont choisis parmi les alcanediols et les polyalkylènediols.

12. Composition de coiffage selon la revendication 11, **caractérisée par le fait que** les diols formant les motifs (b) sont choisis parmi l'éthylèneglycol, le propylèneglycol, le diéthylèneglycol, le triéthylèneglycol et le polypropylèneglycol.

13. Composition de coiffage selon l'une quelconque des revendications 4 à 12, **caractérisée par le fait que** les diamines formant les motifs (b) sont choisies parmi les alcanediamines et les poly(oxyalkylène)diamines.

14. Composition de coiffage selon l'une quelconque des revendications 4 à 13, **caractérisée par le fait que** le monomère (c) portant au moins une fonction sulfonique est choisi parmi les acides dicarboxyliques, les esters d'acides dicarboxyliques, les glycols et les hydroxyacides, portant tous au moins un groupe sulfonique, sous forme acide et/ou neutralisée, de préférence sous forme neutralisée.

15. Composition de coiffage selon l'une quelconque des revendications 4 à 14, **caractérisée par le fait que** le monomère multifonctionnel (d) est choisi parmi le triméthyloléthane, le triméthylolpropane, le glycérol, le pentaérythritol, le sorbitol, l'anhydride trimellitique, l'érythritol, le thréitol, le dipentaérythritol, le dianhydride pyromellitique et l'acide diméthylpropionique.

16. Composition de coiffage selon l'une quelconque des revendications précédentes, **caractérisée par le fait que** le polyester sulfonique ramifié représente de 0,2 à 15%, et de préférence de 0,5 à 10% du poids total de la phase liquide de la composition de coiffage.

17. Composition de coiffage selon l'une quelconque des revendications précédentes, **caractérisée par le fait que** la teneur en eau de la phase liquide est comprise entre 70 % et 98 %, de préférence entre 75 et 96 % en poids.

18. Composition de coiffage selon l'une quelconque des revendications précédentes, **caractérisée par le fait qu'**il s'agit d'une laque pour cheveux.

19. Procédé de coiffage par pulvérisation d'une composition de coiffage, conditionnée dans un dispositif aérosol, selon l'une quelconque des revendications précédentes, sur les cheveux.

## Claims

1. Styling composition packaged in an aerosol device, comprising
• from 45% to 65% by weight of a liquid phase containing, in dissolved or finely dispersed form in a cosmetically acceptable aqueous or aqueous-alcoholic liquid medium, at least one branched sulfonic polyester, and
• from 35% to 55% by weight of dimethyl ether as propellant,
the total water content of the liquid phase being between 65% and 99% by weight.

2. Styling composition according to Claim 1, **characterized in that** it contains ethanol and **in that** the ethanol/dimethyl ether weight ratio is less than or equal to 1.

3. Styling composition according to Claim 2, **characterized in that** the liquid phase has an ethanol content of less than or equal to 35% by weight, preferably less than or equal to 25% by weight and in particular less than or equal to 15% by weight.

4. Styling composition according to any one of the preceding claims, **characterized in that** the branched sulfonic polyester is a polymer formed by polycondensation
(a) of at least one dicarboxylic acid not bearing a sulfonic function,
(b) of at least one diol or a mixture of a diol and a diamine,
(c) of at least one monomer comprising two reactive functions, which may be identical or different, chosen from hydroxyl, amino and carboxyl groups, and also bearing at least one sulfonic function, and
(d) of at least one monomer comprising at least three reactive functions, which may be identical or different, chosen from hydroxyl, amino and carboxyl groups.

5. Styling composition according to Claim 4, **characterized in that** the branched sulfonic polyester also contains units derived from difunctional monomers (e) chosen from hydroxy carboxylic acids and amino carboxylic acids, or a mixture thereof.

6. Styling composition according to Claim 5, **characterized in that** the difunctional monomer (e) represents up to 40 mol% of the total amount of monomers.

7. Styling composition according to one of Claims 4 to 6, **characterized in that** the monomer (c) bearing at least one sulfonic function represents from 2 mol% to 15 mol% of the total amount of monomers.

8. Styling composition according to any one of Claims 4 to 7, **characterized in that** the monomer (d) bearing at least three reactive functions represents from 0.1 mol% to 40 mol% of the total amount of monomers.

9. Styling composition according to any one of Claims 4 to 8, **characterized in that** the dicarboxylic acids not bearing a sulfonic function forming the units (a) are chosen from aliphatic dicarboxylic acids, alicyclic dicarboxylic acids and aromatic dicarboxylic acids, and mixtures of such acids.

10. Styling composition according to Claim 9, **characterized in that** the dicarboxylic acids forming the units (a) are chosen from the group formed by 1,4-cyclohexanedioic acid, succinic acid, glutaric acid, adipic acid, azelaic acid, sebacic acid, fumaric acid, maleic acid, 1,3-cyclohexanedioic acid, phthalic acid, terephthalic acid and isophthalic acid, and a mixture of such acids.

11. Styling composition according to any one of Claims 4 to 10, **characterized in that** the diols forming the units (b) are chosen from alkanediols and polyalkylenediols.

12. Styling composition according to Claim 11, **characterized in that** the diols forming the units (b) are chosen from ethylene glycol, propylene glycol, diethylene glycol, triethylene glycol and polypropylene glycol.

13. Styling composition according to any one of Claims 4 to 12, **characterized in that** the diamines forming the units (b) are chosen from alkanediamines and poly(oxyalkylene)diamines.

14. Styling composition according to any one of Claims 4 to 13, **characterized in that** the monomer (c) bearing at least one sulfonic function is chosen from dicarboxylic acids, dicarboxylic acid esters, glycols and hydroxy acids, all bearing at least one sulfonic group, in acid and/or neutralized form, preferably in neutralized form.

15. Styling composition according to any one of Claims 4 to 14, **characterized in that** the multifunctional monomer (d) is chosen from trimethylolethane, trimethylolpropane, glycerol, pentaerythritol, sorbitol, trimellitic anhydride, erythritol, threitol, dipentaerythritol, pyromellitic dianhydride and dimethylpropionic acid.

16. Styling composition according to any one of the preceding claims, **characterized in that** the branched sulfonic polyester represents from 0.2% to 15% and preferably from 0.5% to 10% of the total weight of the liquid phase of the styling composition.

17. Styling composition according to any one of the preceding claims, **characterized in that** the water content of the liquid phase is between 70% and 98% and preferably between 75% and 96% by weight.

18. Styling composition according to any one of the preceding claims, **characterized in that** it is a hair lacquer.

19. Process for styling by spraying a styling composition, packaged in an aerosol device, according to any one of the preceding claims, onto the hair.

## Patentansprüche

1. Zusammensetzung für die Frisurengestaltung, die in einer Aerosolvorrichtung konfektioniert ist und enthält
• 45 bis 65 Gew.-% einer flüssigen Phase, die in gelöster Form oder fein verteilt in einem kosmetisch akzeptablen, wässrigen oder wässrig-alkoholischen, flüssigen Medium mindestens einen verzweigten Sulfonsäurepolyester enthält, und
• 35 bis 55 Gew.-% Dimethylether als Treibmittel,
wobei die Gesamtwassermenge der flüssigen Phase im Bereich von 65 bis 99 Gew.-% liegt.

2. Zusammensetzung für die Frisurengestaltung nach Anspruch 1, **dadurch gekennzeichnet, dass** sie Ethanol enthält, und dadurch, dass das Gewichtsverhältnis Ethanol/ Dimethylether kleiner oder gleich 1 ist.

3. Zusammensetzung für die Frisurengestaltung nach Anspruch 2, **dadurch gekennzeichnet, dass** die flüssige Phase einen Ethanolgehalt von 35 Gew.-% oder darunter, vorzugsweise 25 Gew.-% oder darunter und insbesondere 15 Gew.-% oder darunter aufweist.

4. Zusammensetzung für die Frisurengestaltung nach einem der vorhergehenden Ansprüche, **dadurch gekennzeichnet, dass** der verzweigte Sulfonsäurepolyester ein Polymer ist, das gebildet wird durch Polykondensation
(a) mindestens einer Dicarbonsäure, die keine Sulfonsäurefunktion aufweist,
(b) mindestens eines Diols oder eines Gemisches aus einem Diol und einem Diamin,
(c) mindestens eines Monomers, das zwei reaktive Funktionen aufweist, die gleich oder verschieden sind und die unter den Gruppen Hydroxy, Amino und Carboxy ausgewählt sind, und ferner mindestens eine Sulfonsäurefunktion trägt, und
(d) mindestens eines Monomers, das mindestens drei reaktive Funktionen aufweist, die gleich oder verschieden sind und unter den Gruppen Hydroxy, Amino und Carboxy ausgewählt sind.

5. Zusammensetzung für die Frisurengestaltung nach Anspruch 4, **dadurch gekennzeichnet, dass** der verzweigte Sulfonsäurepolyester ferner Einheiten aufweist, die von difunktionellen Monomeren (e) abgeleitet sind, die unter den Hydroxycarbonsäuren und Aminocarbonsäuren oder einem Gemisch aus diesen ausgewählt sind.

6. Zusammensetzung für die Frisurengestaltung nach Anspruch 5, **dadurch gekennzeichnet, dass** das difunktionelle Monomer (e) bis zu 40 Mol-% der gesamten Monomere ausmacht.

7. Zusammensetzung für die Frisurengestaltung nach einem der Ansprüche 4 bis 6, **dadurch gekennzeichnet, dass** das Monomer (c), das mindestens eine Sulfonsäurefunktion aufweist, 2 bis 15 Mol-% der gesamten Monomere ausmacht.

8. Zusammensetzung für die Frisurerigestaltung nach einem der Ansprüche 4 bis 7, **dadurch gekennzeichnet, dass** das Monomer (d), das mindestens drei reaktive Funktionen aufweist, 0,1 bis 40 Mol-% der gesamten Monomere ausmacht.

9. Zusammensetzung für die Frisurengestaltung nach einem der Ansprüche 4 bis 8, **dadurch gekennzeichnet, dass** die Dicarbonsäuren, die keine Sulfonsäurefunktion aufweisen und die die Einheiten (a) bilden, unter den aliphatischen Dicarbonsäuren, alicyclischen Dicarbonsäuren, aromatischen Dicarbonsäuren und Gemischen solcher Säuren ausgewählt sind.

10. Zusammensetzung für die Frisurengestaltung nach Anspruch 9, **dadurch gekennzeichnet, dass** die Dicarbonsäuren, die die Einheiten (a) bilden, unter 1,4-Cyclohexandisäure, Bernsteinsäure, Glutarsäure, Adipinsäure, Azelainsäure, Sebacinsäure, Fumarsäure, Maleinsäure, 1,3-Cyclohexandisäure, Phthalsäure, Terephthalsäure und Isophthalsäure und einem Gemisch dieser Säuren ausgewählt sind.

11. Zusammensetzung für die Frisurengestaltung nach einem der Ansprüche 4 bis 10, **dadurch gekennzeichnet, dass** die Diole, die die Einheiten (b) bilden, unter den Alkandiolen und Polyalkylendiolen ausgewählt sind.

12. Zusammensetzung für die Frisurengestaltung nach Anspruch 11, **dadurch gekennzeichnet, dass** die Diole, die die Einheiten (b) bilden, unter Ethylenglycol, Propylenglycol, Diethylenglycol, Triethylenglycol und Polypropylenglycol ausgewählt sind.

13. Zusammensetzung für die Frisurengestaltung nach einem der Ansprüche 4 bis 12, **dadurch gekennzeichnet, dass** die Diamine, die die Einheiten (b) bilden, unter den Alkandiaminen und Poly(oxyalkylen)diaminen ausgewählt sind.

14. Zusammensetzung für die Frisurengestaltung nach einem der Ansprüche 4 bis 13, **dadurch gekennzeichnet, dass** das Monomer (c), das mindestens eine Sulfonsäurefunktion aufweist, unter den Dicarbonsäuren, Dicarbonsäureestern, Glycolen und Hydroxysäuren ausgewählt ist, die alle mindestens eine Sulfonsäuregruppe in Form der Säure und/oder in neutralisierter Form und vorzugsweise in neutralisierter Form tragen.

15. Zusammensetzung für die Frisurengestaltung nach einem der Ansprüche 4 bis 14, **dadurch gekennzeichnet, dass** das multifunktionelle Monomer (d) unter Trimethylolethan, Trimethylolpropan, Glycerol, Pentaerythritol, Sorbitol, Trimellitsäureanhydrid, Erythritol, Threitol, Dipentaerythritol, Pyromellitsäuredianhydrid und Dimethylpropionsäure ausgewählt ist.

16. Zusammensetzung für die Frisurengestaltung nach einem der vorhergehenden Ansprüche, **dadurch gekennzeichnet, dass** der verzweigte Sulfonsäurepolyester 0,2 bis 15 % und vorzugsweise 0,5 bis 10 % des Gesamtgewichts der flüssigen Phase der Zusammensetzung für die Frisurengestaltung ausmacht.

17. Zusammensetzung für die Frisurengestaltung nach einem der vorhergehenden Ansprüche, **dadurch gekennzeichnet, dass** der Wassergehalt der flüssigen Phase im Bereich von 70 bis 98 Gew.-% und vorzugsweise 75 bis 96 Gew.-% liegt.

18. Zusammensetzung für die Frisurengestaltung nach einem der vorhergehenden Ansprüche, **dadurch gekennzeichnet, dass** es sich um einen Haarlack handelt.

19. Verfahren für die Frisurengestaltung durch Zerstäuben einer Zusammensetzung für die Frisurengestaltung nach einem der vorhergehenden Ansprüche, die in einer Aerosolvorrichtung konfektioniert ist, auf den Haaren.
